# EUROPEAN PATENT APPLICATION

(11) **EP 2 215 985 A1**
(43) Date of publication of application: **11.08.2010**
(21) Application number: 08845018.4
(22) Date of filing: 31.10.2008
(51) Int. Cl.: A61B 18/02

(54) **CRYOTHERAPY DEVICE AND PROBE FOR CRYOTHERAPY**

(30) Priority: 02.11.2007 JP 2007286595
(71) Applicant: National University Corporation Hamamatsu University School of Medicine, Hamamatsu-shi, Shizuoka 431-3192 (JP); Twinbird Corporation, Niigata 959-0292 (JP)
(72) Inventor: ISODA, Haruo, Hamamatsu-shi Shizuoka 431-3192 (JP); SAKAHARA, Harumi, Hamamatsu-shi Shizuoka 431-3192 (JP); FUJINO, Hitoshi, Tsubame-shi Niigata 959-0292 (JP); SUZUKI, Takeshi, Tsubame-shi Niigata 959-0292 (JP)
(74) Representative: Harris, Ian Richard
(86) International application number: PCT/JP2008/069955
(87) International publication number: WO 2009/057779

(57) **Abstract**

In a cryotherapy apparatus 1, an FPSC2 (free piston type Stirling cooling machine) is used that is driven by a normal power supply, and that is comparatively small and inexpensive. In addition, to a heat absorbing portion 2a of the FPSC2 connected is a base end 6a of an inner pipe 6 in which a refrigerant that operates at a low temperature is encapsulated. As a result of this, when the FPSC2 is driven in a state where a heat exchanging portion 8 provided at a tip 6b of the inner pipe 6 is located on a predetermined site inside a body, heat moves from the heat exchanging portion 8 to the heat absorbing portion 2a of the FPSC2 through the refrigerant. At this time, the refrigerant encapsulated in the inner pipe 6 is insulated from the outside since a vacuumed gap S is formed by the inner pipe 6 being covered with an outer pipe 7. As a result of this, an ice ball is formed on the predetermined site where the heat exchanging portion 8 has been located.

## Description

### Technical Field

The present invention relates to a cryotherapy apparatus and a cryotherapy probe that freeze-treat a site with a deep-seated neoplastic lesion etc.

### Background Art

In recent years, a cryotherapy probe utilizing a Joule Thomson effect with argon gas has been developed, and a site with a deep-seated neoplastic lesion can be accurately freeze-treated under MRI guidance (for example, refer to Non-Patent Document 1). In addition, a conventional thermosiphon is widely used for heating, little used for cooling to a low temperature, and there is no example that achieves freezing at less than -20 degrees C as an apparatus for freeze-treating a deep-seated lesion. Further, in cryotherapy of the deep-seated lesion, there has never been a thermosiphon that cycle-controls freezing and thawing. Particularly, in cryotherapy of a deep-seated tumor, it is important for improving an effect of treatment that a thermosiphon is made to reach a deep-seated affected site of a body from an outside thereof, and that freezing and thawing are repeated on the affected site in a limited and focused way, but it has been impossible to treat a target affected site by freezing and thawing in a cycle-controlled way using the conventional thermosiphon.
Non-patent Document 1
"4. A new development of cryotherapy with MRI" written by Harada Junta and the others in p.12 to 14 of May and June issues in 2003 of monthly Inner Vision

### Disclosure of the Invention

However, there has been a problem in the cryotherapy apparatus utilizing the Joule Thomson effect that the apparatus is large and expensive. Further, there has also been a problem that the apparatus is complicated to be handled, for example, a particular room is required since a high-pressure gas cylinder of 300 atmospheres etc. is used, or it is necessary to newly prepare not only a cryotherapy probe but a high pressure gas for every use. An additional important problem to be solved of the present invention is to form and present a state where freezing and thawing that is extremely effective in cryotherapy of a deep-seated lesion can be cycle-controlled in a limited and focused way with respect to an affected site.

Consequently, the present invention is made in view of such situations, and aims at providing a cryotherapy apparatus and a cryotherapy probe that can achieve facilitation of handling, reduction in size, and price-reduction.

In order to achieve the aforementioned object, a cryotherapy apparatus in accordance with the present invention is a cryotherapy apparatus for freeze-treating a predetermined site inside a body, and it is provided with a Stirling cooling machine and a cryotherapy probe attached to a heat absorbing portion of this Stirling cooling machine, and it is **characterized in that** this cryotherapy probe has an inner pipe whose base end is connected to the heat absorbing portion as well as in which a refrigerant that operates below the freezing point is encapsulated, an outer pipe that covers the inner pipe so that a gap to be vacuumed may be formed, and a heat exchanging portion provided at a tip of the inner pipe.

In this cryotherapy apparatus, a Stirling cooling machine is used that is generally driven by normal power supply and that is comparatively small and inexpensive (for example, free piston type Stirling cooling machine). In addition, the base end of the inner pipe in which the refrigerant has been encapsulated is connected to the heat absorbing portion of this Stirling cooling machine. As a result of this, when the Stirling cooling machine is driven in a state where the heat exchanging portion provided at the tip of the inner pipe is located at a predetermined site inside a body, heat moves from the heat exchanging portion of the cryotherapy probe to the heat absorbing portion of the Stirling cooling machine through the refrigerant. At this time, the refrigerant encapsulated in this inner pipe is insulated from the outside since the outer pipe covers the inner pipe so that the gap to be vacuumed may be formed. Hence, an ice ball can be efficiently formed on a predetermined site where the heat exchanging portion is located, thus enabling to freeze-treat the predetermined site. As described above, this cryotherapy apparatus can achieve facilitation of handling, reduction in size, and price-reduction.

It is preferable for a cryotherapy apparatus in accordance with the present invention to provide a heater that heats the heat exchanging portion or the heat absorbing portion, and to provide a temperature sensor that detects a temperature of the heat exchanging portion or the heat absorbing portion, and it is more preferable for the Stirling cooling machine to be a free piston type Stirling cooling machine, and for the cryotherapy apparatus to provide a control unit that switches drive of the free piston type Stirling cooling machine and drive of the heater based on a temperature detected by the temperature sensor. In this case, freezing and thawing can be repeated with respect to a predetermined site inside a body, thus enabling to freeze-treat the predetermined site more reliably.

In the cryotherapy apparatus in accordance with the present invention, it is preferable for the heat exchanging portion to be removable with respect to the inner pipe. In this case, it becomes possibly to use various types of the heat exchanging portions depending on a state of the predetermined site targeted for cryotherapy.

In the cryotherapy apparatus in accordance with the present invention, it is preferable for the cryotherapy probe to be removable with respect to the heat absorbing portion. In this case, it becomes possible to exchange the cryotherapy probe for a new one or to disinfect the cryotherapy probe before use.

In the cryotherapy apparatus in accordance with the present invention, it is preferable that the outer pipe and the heat exchanging portion are made of stainless steel or titanium. In this case, it becomes possible to improve rust proof performance of the outer pipe and the heat exchanging portion that are inserted in the body and that are exposed outside.

In the cryotherapy apparatus in accordance with the present invention, it is preferable that the Stirling cooling machine is supported by a free-arm supporting mechanism. In this case, it becomes possible to handle the cryotherapy apparatus more easily in a stable state.

In the cryotherapy apparatus in accordance with the present invention, it is preferable that the heat absorbing portion is housed in a chamber to be vacuumed, and that the outer pipe is airtightly connected to the chamber to be opened to an inside of this chamber in the state' where the base end of the inner pipe is connected to the heat absorbing portion, and it is preferable for the cryotherapy apparatus in accordance with the present invention to provide a vacuum pump that is connected to the chamber and that vacuums the inside of this chamber and a gap between the inner pipe and the outer pipe. In this case, the heat absorbing portion and the refrigerant encapsulated in the inner pipe can be reliably insulated from the outside as well as the inside of the chamber and the gap between the inner pipe and the outer pipe can be efficiently vacuumed.

In the cryotherapy apparatus in accordance with the present invention, it is preferable that a core made of a material whose heat conductivity is higher than this heat exchanging portion is embedded in the heat exchanging portion, and that this core extends inside the tip of the inner pipe. In this case, even if the heat exchanging portion is made thin in order to make it correspond to a predetermined site inside the body, reliable transfer of the heat can be achieved in the heat exchanging portion through the core extended inside the tip of the inner pipe in which the refrigerant has been encapsulated.

In addition, a cryotherapy probe in accordance with the present invention is a cryotherapy probe used for a cryotherapy apparatus for freeze-treating a predetermined site inside a body, and it has an inner pipe in which a refrigerant that operates below the freezing point has been encapsulated, an outer pipe that covers the inner pipe so that a gap to be vacuumed may be formed, and a heat exchanging portion provided at a tip of the inner pipe, and it is **characterized in that** a core made of a material whose heat conductivity is higher than this heat exchanging portion is embedded in this heat exchanging portion, and that this core extends inside the tip of the inner pipe.

As described above, this cryotherapy probe can achieve reliable transfer of the heat in the heat exchanging portion through the core extended inside the tip of the inner pipe in which the refrigerant has been encapsulated.

### Effect of the Invention

The present invention can achieve facilitation of handling, reduction in size, and price-reduction. When ease of handling improves, it becomes possible to achieve reduction of an operation time and reduction of a patient burden in an operation.

### Brief Description of the Drawings

Figure 1 is a configuration diagram of a first embodiment of a cryotherapy apparatus in accordance with the present invention;
Figure 2 is a sectional view of an FPSC side portion in a cryotherapy probe of the cryotherapy apparatus of Figure 1;
Figure 3 is a sectional view of a portion opposite to the FPSC in the cryotherapy probe of the cryotherapy apparatus of Figure 1;
Figure 4 is a block diagram of a control unit of the cryotherapy apparatus of Figure 1;
Figure 5 is a timing chart of the control unit of the cryotherapy apparatus of Figure 1;
Figure 6 is a configuration diagram of a second embodiment of the cryotherapy apparatus in accordance with the present invention;
Figure 7 is a sectional view of an FPSC side portion in a cryotherapy probe of the cryotherapy apparatus of Figure 6;
Figure 8 is a sectional view of a portion opposite to the FPSC in the cryotherapy probe of the cryotherapy apparatus of Figure 6;
Figure 9 is a block diagram of a control unit of the cryotherapy apparatus of Figure 6; and
Figure 10 is a sectional view of a portion opposite to an FPSC in a cryotherapy probe of another embodiment of the cryotherapy apparatus in accordance with the present invention.

### Description of Symbols

1 ... Cryotherapy apparatus, 2 ... FPSC, 2a ... Heat absorbing portion, 3 ... Cryotherapy probe, 4 ... Supporting mechanism, 6 ... Inner pipe, 6a ... Base end, 6b ... Tip, 7 ... Outer pipe, 8 ... Heat exchanging portion, 12 ... Heater, 14 ... Temperature sensor, 20 ... Control unit, 31 ... Vacuum pump, 32 ... Core, 34... Chamber.

### Best Modes for Carrying Out the Invention

Hereinafter, preferred embodiments of the present invention will be explained in detail with reference to drawings. It is to be noted that the same reference numeral is given to the same or a corresponding part in each drawing, and a duplicated explanation will be omitted.

### [First embodiment]

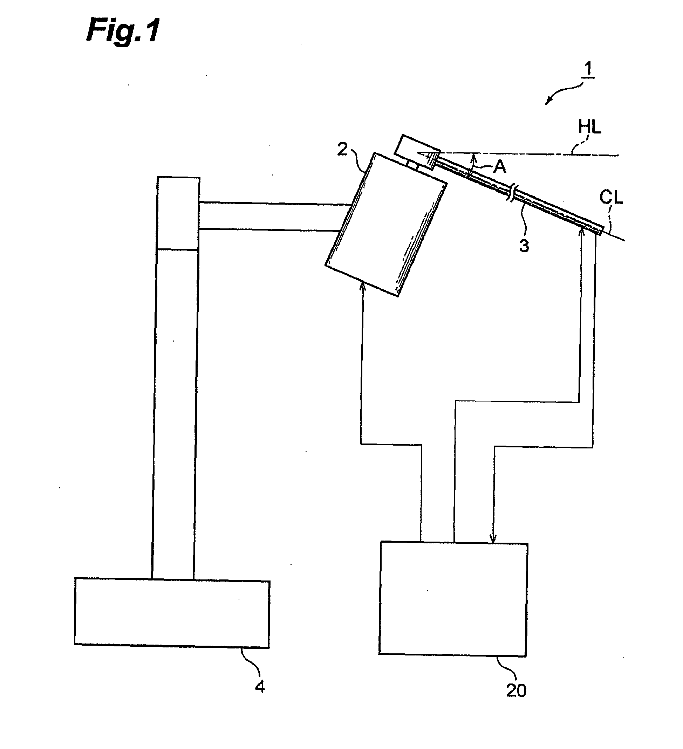

Figure 1 is a configuration diagram of a first embodiment of a cryotherapy apparatus in accordance with the present invention. As shown in Figure 1, a cryotherapy apparatus 1 is provided with a free piston type Stirling cooling machine (hereinafter refer to as "FPSC") 2, a cryotherapy probe 3 (tubular body as a thermosiphon) attached to a heat absorbing portion of this FPSC2, a free-arm supporting mechanism 4 that rotatably supports the FPSC2, and a control unit 20, such as a personal computer. The cryotherapy apparatus 1 is an apparatus for freeze-treating a site with a deep-seated neoplastic lesion under image guidance using MRI, CT, an ultrasonic diagnostic equipment, etc. It is to be noted that an FPSC with a well-known configuration is used as the FPSC2 (for example, refer to Japanese Patent Laid-Open No. 2005-337551).

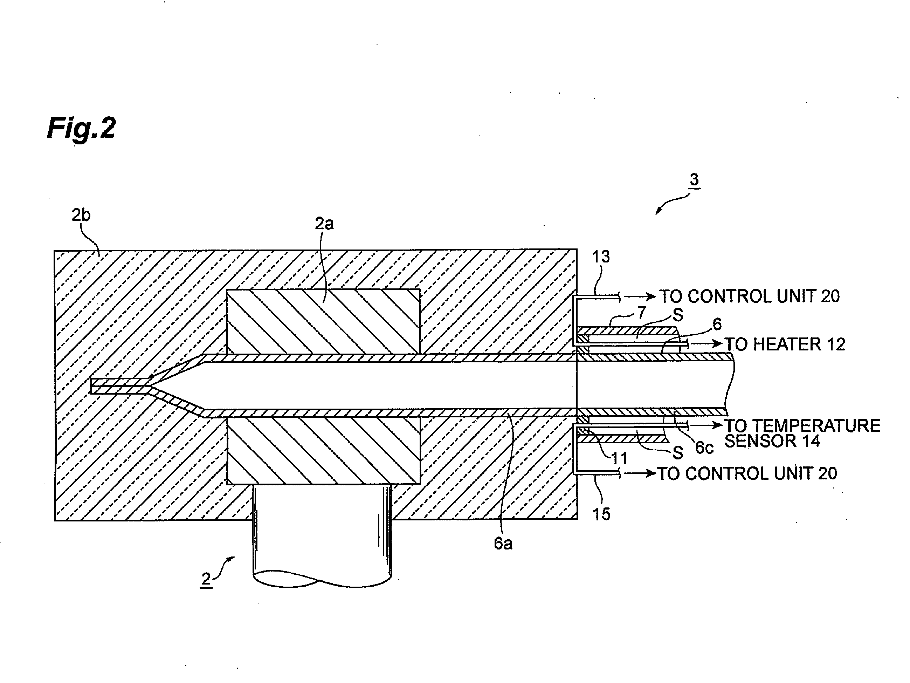

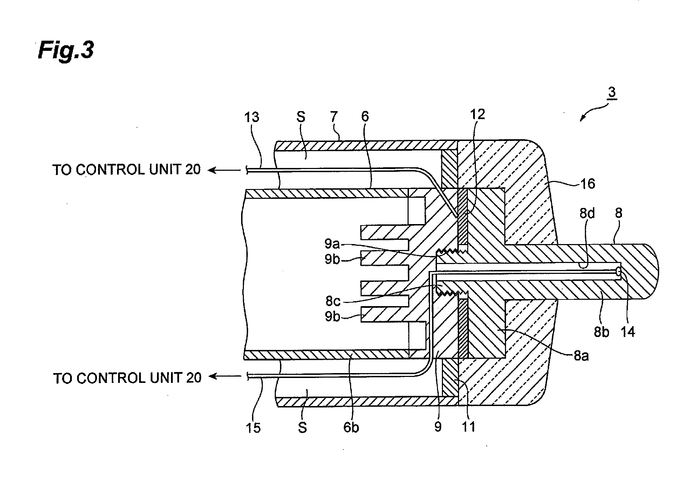

Figure 2 is a sectional view of the FPSC2 side portion in the cryotherapy probe 3 of the cryotherapy apparatus 1 of Figure 1, and Figure 3 is a sectional view of a portion opposite to the FPSC2 in the cryotherapy probe 3 of the cryotherapy apparatus 1 of Figure 1. As shown in Figures 2 and 3, the cryotherapy probe 3 has an inner pipe 6 whose base end 6a is connected to a heat absorbing portion 2a of the FPSC2, an outer pipe 7 that covers a tip 6b of the inner pipe 6 and an intermediate portion 6c (portion between the base end 6a and the tip 6b of the inner pipe 6) of the inner pipe 6 so that a gap S may be formed, and a heat exchanging portion 8 provided at the tip 6b of the inner pipe 6. The base end 6a is inserted in the heat absorbing portion 2a, whereby the cryotherapy probe 3 is attached to this heat absorbing portion 2a. That is, the cryotherapy probe 3 is removable with respect to the heat absorbing portion 2a. It is to be noted that the heat absorbing portion 2a and the base end 6a of the inner pipe 6 are covered with a heat insulating material 2b. In addition, an end of the tip 6b of the inner pipe 6 need not be covered with the outer pipe 7.

In the inner pipe 6, the tip 6b and the intermediate portion 6c are cylindrical pipes made of stainless steel (for example, SUS304), and the base end 6a is a cylindrical pipe made of copper. The tip 6b and the intermediate portion 6c are integrally formed, and the base end 6a and the intermediate portion 6c are airtightly connected by welding such as brazing. An attaching portion 9 made of stainless steel (for example, SUS304) is airtightly connected to the tip 6b of the inner pipe 6 by welding such as brazing.

A refrigerant, such as carbon dioxide, chlorofluorocarbon, or naphthalene, is encapsulated in the inner pipe 6 at a pressure of approximately 10 to 50 atmospheres. This encapsulation of the refrigerant is performed by crushing the base end 6a with a pinch and airtightly connecting the crushed portion by welding such as brazing after injecting the refrigerant in the inner pipe 6. It is to be noted that there can be improved an efficiency of heat exchange of the heat absorbing portion 2a of the FPSC2 and the refrigerant in the inner pipe 6 as well as welding, such as a pinch and brazing, can be easily performed since the base end 6a is made of copper. It is to be noted that heat conduction is sacrificed a little, but it is also possible to integrally process the whole inner pipe 6 with a stainless steel pipe.

The gap S formed between the inner pipe 6 and the outer pipe 7 is vacuumed. In a vacuum chamber, the inner pipe 6 to which the attaching portion 9 is connected is arranged in the outer pipe 7, and an insulating member 11 made of silicone resin, a foamed rubber, or the like is airtightly connected with adhesive between the inner pipe 6 to which the attaching portion 9 is connected and both ends of the outer pipe 7, whereby this vacuuming is performed.

The heat exchanging portion 8 has a disk-like body 8a, a cylindrical projecting portion 8b that extends from this body 8a to a tip side, and a screw portion 8c that extends from the body 8a to a base end side. The body 8a, the projecting portion 8b, and the screw portion 8c are made of stainless steel (for example, SUS304), and are formed integrally. At the heat exchanging portion 8, formed is a narrow hole 8d that passes through the body 8a from the base end side of the screw portion 8c to the tip of the projecting portion 8b. The screw portion 8c is screwed to a screw hole 9a formed at a tip side of the attaching portion 9, whereby the heat exchanging portion 8 is provided at the tip 6b of the inner pipe 6. That is, the heat exchanging portion 8 is removable with respect to the inner pipe 6. It is to be noted that an efficiency of heat exchange of the refrigerant in the inner pipe 6 and the heat exchanging portion 8 can be improved since a plurality of fins 9b are formed at a rear end side of the attaching portion 9.

A heater 12, such as a ceramic heater or a PTC heater, is attached to the attaching portion 9 so as to contact the body 8a of the heat exchanging portion 8. A nichrome wire or the like may be wound around the attaching portion 9 as this heater 12. That is, the heater 12 is to heat the heat exchanging portion 8. In addition, the heater 12 is connected to the control unit 20 through a wire 13 that passes through the gap S to be pulled out from a rear end side thereof. A temperature sensor 14 such as a thermistor is attached inside the narrow hole 8d of the heat exchanging portion 8 so as to contact a bottom thereof. That is, this temperature sensor 14 is to detect a temperature of a tip of the projecting portion 8b of the heat exchanging portion 8. Further, the temperature sensor 14 is connected to the control unit 20 through a wire 15 that passes through the gap S to be pulled out from the rear end side thereof. It is to be noted that a portion excluding the tip of the projecting portion 8b in the heat exchanging portion 8 and the heater 12 are covered with an insulating member 16 made of silicone resin, a foamed rubber, or the like.

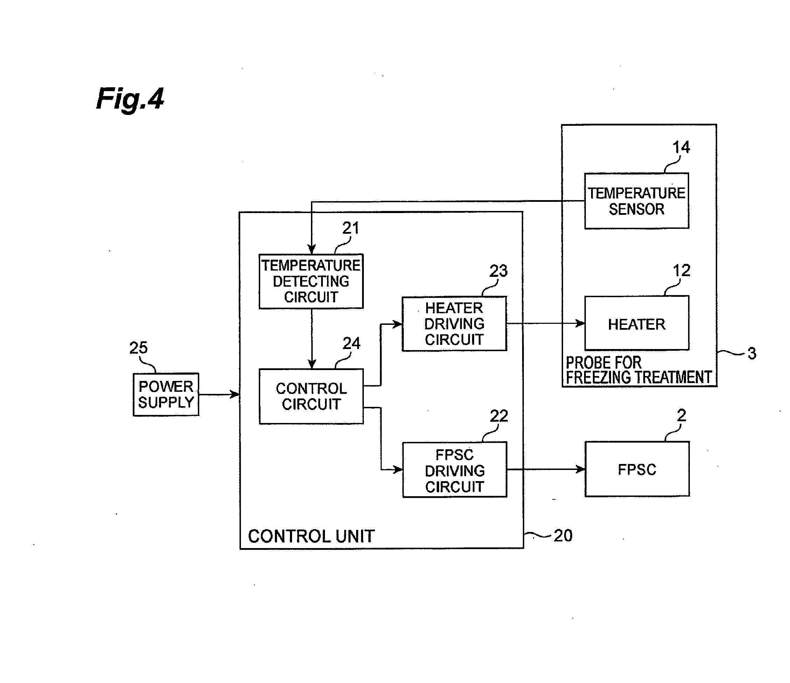

Figure 4 is a block diagram of the control unit of the cryotherapy apparatus 1 of Figure 1. As shown in Figure 4, the control unit 20 has a temperature detecting circuit 21 that obtains a temperature signal showing a temperature detected by the temperature sensor 14, an FPSC driving circuit 22 that transmits a drive signal to the FPSC2, a heater driving circuit 23 that transmits a drive signal to the heater 12, and a control circuit 24 that instructs the FPSC driving circuit 22 or the heater driving circuit 23 to transmit a drive signal based on the temperature signal obtained by the temperature detecting circuit 21. The control unit 20 is connected to a normal power supply 25, and switches drive of the FPSC2 and drive of the heater 12 based on a temperature detected by the temperature sensor 14.

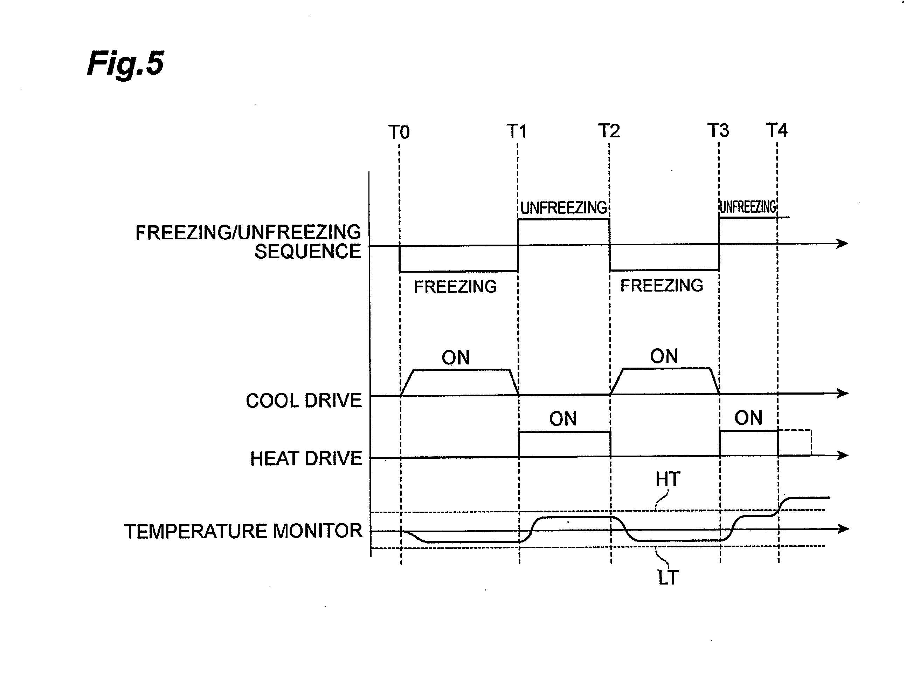

An example of use of the cryotherapy apparatus 1 configured as described above will be explained. Figure 5 is a timing chart of the control unit of the cryotherapy apparatus 1 of Figure 1.

First, the cryotherapy probe 3 is inserted inside a body in a state where the FPSC2 is supported by the supporting mechanism 4, and the tip of the projecting portion 8b of the heat exchanging portion 8 is located on a site with a deep-seated neoplastic lesion under image guidance using MRI, CT, an ultrasonic diagnostic equipment, or the like. At this time, as shown in Figure 1, the FPSC2 is rotated so that a tip of the cryotherapy probe 3 may come down with respect to a horizontal line HL (specifically, an angle A formed by the horizontal line HL and a center line CL of the cryotherapy probe 3 may become 15 to 90 degrees, preferably not less than 20 degrees, and more preferably around 50 degrees), and then supported by the supporting mechanism 4. The reason is that a higher cooling effect brought by the cryotherapy probe 3 can be exerted.

Subsequently, as shown in Figure 5, drive of the FPSC2 (cool drive) and drive of the heater 12 (heat drive) are switched by the control unit 20 based on the temperature detected by the temperature sensor 14 (temperature monitor). Consequently, freezing and thawing of a site with a deep-seated neoplastic lesion are repeated (freezing/thawing sequence), and as a result of this, a tumor necrotizes and the site with the deep-seated neoplastic lesion is freeze-treated. As one of the examples, when a tumor is formed of cancer cells, it is frozen to approximately -20 to -40 °C to form an ice ball of a predetermined size (approximately 5 centimeters in diameter) is formed. Subsequently, this one cycle is performed twice by defining freezing for about 10 minutes and thawing for about 10 minutes as one cycle.

It is to be noted that in order to prevent excessive fall of a temperature by the FPSC2 and excessive rise of a temperature by the heater 12, and to thereby secure safety, an upper limit threshold HT and a lower limit threshold LT are set in the temperature monitor, and if the temperature exceeds the upper limit threshold HT (refer to a timing T4 shown in Figure 5), heat drive is stopped, while if the temperature is below the lower limit threshold LT, cool drive is stopped.

As explained above, in the cryotherapy apparatus 1, the FPSC2 is used that is driven by the normal power supply, and that is comparatively small and inexpensive. In addition, the base end 6a of the inner pipe 6 in which the refrigerant is encapsulated is connected to the heat absorbing portion 2a of this FPSC2. As a result of this, when the FPSC2 is driven in a state where the heat exchanging portion 8 provided at the tip 6b of the inner pipe 6 is located in a predetermined site inside the body, heat moves from the heat exchanging portion 8 of the cryotherapy probe 3 to the heat absorbing portion 2a of the FPSC2 through the refrigerant. At this time, the refrigerant encapsulated in the inner pipe 6 is insulated from the outside since the vacuumed gap S is formed by the inner pipe 6 being covered with the outer pipe 7. Hence, an ice ball can be efficiently formed on the predetermined site where the heat exchanging portion 8 is located. Meanwhile, the heater 12 that heats the heat exchanging portion 8 is provided at the cryotherapy probe 3. As a result of this, when the heater 12 is driven in a state where the heat exchanging portion 8 is located in the predetermined site inside the body, heat moves from the heat exchanging portion 8 of the cryotherapy probe 3 to the predetermined site inside the body. Hence, an ice ball can be efficiently melted formed on the predetermined site where the heat exchanging portion 8 is located. As described above, with the cryotherapy apparatus 1, freezing and thawing can be repeated with respect to a predetermined site inside a body while achieving facilitation of handling, reduction in size, and price-reduction, thus enabling to freeze-treat the predetermined site more reliably.

In addition, in the cryotherapy apparatus 1, the heat exchanging portion 8 is removable with respect to the inner pipe 6. As a result of this, it becomes possible to use various types of heat exchanging portions 8 depending on a state of a predetermined site targeted for cryotherapy.

In addition, in the cryotherapy apparatus 1, the cryotherapy probe 3 is removable with respect to the heat absorbing portion 2a of the FPSC2. As a result of this, it becomes possible to exchange the cryotherapy probe 3 for a new one or to disinfect the cryotherapy probe 3 before use.

In addition, in the cryotherapy apparatus 1, the outer pipe 7 and the heat exchanging portion 8 are made of stainless steel. As a result of this, it becomes possible to improve rust proof performance of the outer pipe 7 and the heat exchanging portion 8 that are inserted in the body and that are exposed outside. In addition to that, it becomes possible to also improve pressure-resisting performance since the tip 6b and the intermediate portion 6c of the inner pipe 6 are also made of stainless steel. It is to be noted that the heat exchanging portion 8 may be made of titanium or duralumin.

Further, in the cryotherapy apparatus 1, the FPSC2 is rotatably supported by the supporting mechanism 4. As a result of this, an angle of the cryotherapy probe 3 can be adjusted so as to exert a higher cooling effect, and it becomes possible to handle the cryotherapy apparatus 1 more easily in a stable state.

### [Second embodiment]

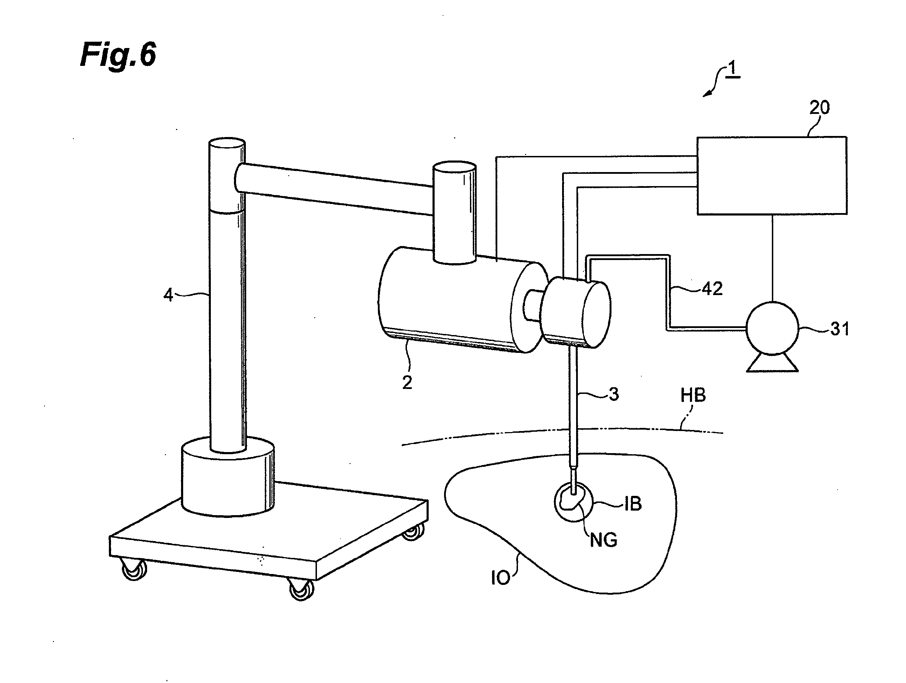

Figure 6 is a configuration diagram of a second embodiment of the cryotherapy apparatus in accordance with the present invention. As shown in Figure 6, the cryotherapy apparatus 1 is provided with the FPSC2, the cryotherapy probe 3 attached to the heat absorbing portion of this FPSC2, the free-arm supporting mechanism 4 that rotatably and movably supports the FPSC2, a vacuum pump 31 that vacuums an inside of the chamber in which the heat absorbing portion is housed in the FPSC2, and the control unit 20 such as a personal computer. The cryotherapy apparatus 1 is an apparatus for freeze-treating a site NG with a deep-seated neoplastic lesion by the following method: the cryotherapy probe 3 is inserted in a human body HB; the tip of the cryotherapy probe 3 is located on the site NG with the deep-seated neoplastic lesion in an organ 10; freezing (formation of an ice ball IB) and thawing of the site NG are repeated; and a tumor is necrotized.

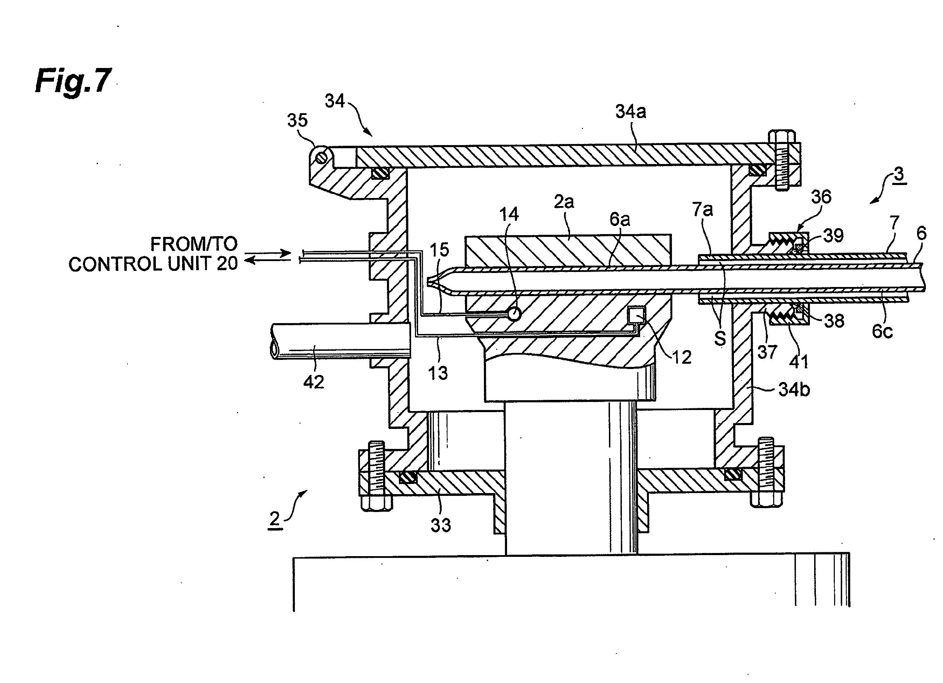

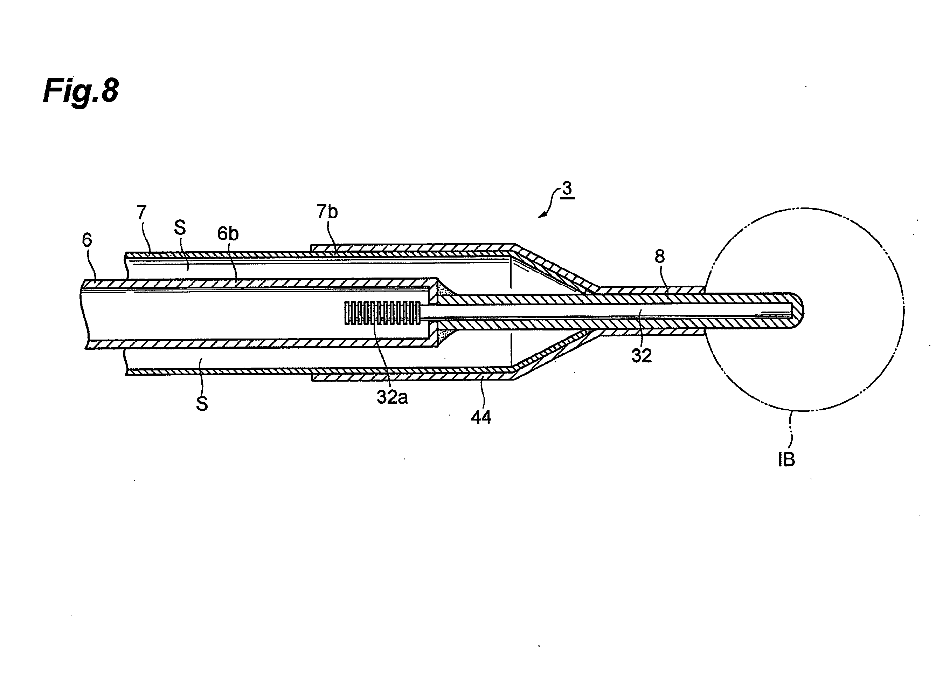

Figure 7 is a sectional view of the FPSC2 side portion in the cryotherapy probe 3 of the cryotherapy apparatus 1 of Figure 6, and Figure 8 is a sectional view of a portion opposite to the FPSC2 in the cryotherapy probe 3 of the cryotherapy apparatus 1 of Figure 6. As shown in Figures 7 and 8, the cryotherapy probe 3 has the inner pipe 6 whose base end 6a is connected to the heat absorbing portion 2a of the FPSC2, the outer pipe 7 that covers the tip 6b and the intermediate portion 6c of the inner pipe 6 so as to form the gap S, and the heat exchanging portion 8 provided at the tip 6b of the inner pipe 6.

The inner pipe 6 is a cylindrical pipe made of copper with 6 to 8 millimeters in outer diameter, and the heat exchanging portion 8 is connected to the tip 6b of the inner pipe 6 by welding such as brazing. A refrigerant is encapsulated in the inner pipe 6 at a pressure of approximately 50 atmospheres. This encapsulation of the refrigerant is performed by crushing the base end 6a with a pinch and airtightly connecting the crushed portion by welding such as brazing after injecting the refrigerant in the cylindrical pipe in which the tip 6b is closed.

It is to be noted that as the refrigerant, carbon dioxide, chlorofluorocarbon alternative, or the like is used in a mixed state of a vapor phase and a liquid phase. If it is sufficient to freeze to -50°C, carbon dioxide is used, and if it is necessary to freeze to -80°C, chlorofluorocarbon alternative is used. Further, if it is necessary to freeze to a temperature not more than that, such as -100°C, natural gas, such as ethylene gas or butane gas, is used.

The outer pipe 7 is a cylindrical pipe made of stainless steel (for example, SUS304) with 9 to 10 millimeters in outer diameter. A base end 7a of the outer pipe 7 is opened in a state where the gap S is maintained. Meanwhile, a tip 7b of the outer pipe 7 is reduced in diameter, and airtightly connected to a side surface of the heat exchanging portion 8 by welding such as brazing.

As shown in Figure 8, the heat exchanging portion 8 is a cylindrical member made of stainless steel (for example, SUS304) with 2 to 3 millimeters in outer diameter and with 20 to 30 millimeters in length, and the outer diameter of the heat exchanging portion 8 is made smaller than that of the inner pipe 6. A rod-like core 32 made of copper is embedded in the heat exchanging portion 8 along a center line thereof. A tip of this core 32 extends to the tip of the heat exchanging portion 8, and is formed hemispherically. Meanwhile, a rear end of the core 32 extends inside the tip 6b of the inner pipe 6, and a plurality of projections are formed on an extended portion 32a in order to make a surface area increase and to improve heat exchange performance.

It is to be noted that a material of the core 32 is not limited to copper as long as it has higher heat conductivity than the heat exchanging portion 8. For example, if the material of the heat exchanging portion 8 is a medical metal, such as stainless steel or titanium, copper, aluminum, silver, alloy thereof, carbon, or the like is used as the material of the core 32. In addition, on a portion excluding a portion where the ice ball IB is assumed to be formed in the heat exchanging portion 8, on a joint portion of the tip 7b of the outer pipe 7 and the side surface of the heat exchanging portion 8, and on a portion of the tip 7b of the outer pipe 7 corresponding to the rear end of the core 32, applied is a heat insulating coat 44 made of Teflon (registered trademark), a silicone rubber, or the like in order to prevent heat from flowing in from the base end 7a of the outer pipe 7 and to improve an efficiency of forming the ice ball IB.

As shown in Figure 7, in the FPSC2, the heat absorbing portion 2a is housed in a chamber 34 airtightly configured on a vacuum flange 33. A ceiling wall 34a of this chamber 34 is openable through a hinge 35, and on a side wall 34b of the chamber 34 attached is a vacuum joint 36 for airtightly connecting the outer pipe 7 of the cryotherapy probe 3 to the side wall 34b of the chamber 34.

The vacuum joint 36 has a cylindrical inserting portion 37 integrally formed on the side wall 34b of the chamber 34, a circular pressing member 38 opposed to this inserting portion 37, an O-ring 39 that contacts a side surface of the outer pipe 7, a tapered surface of the inserting portion 37, and a tapered surface of the pressing member 38, and a cap nut 41 that covers the pressing member 38 and the O-ring 39 to be screwed to the inserting portion 37. The outer pipe 7 of the cryotherapy probe 3 is airtightly connected to the side wall 34b of the chamber 34 by tightening the cap nut 41 onto the inserting portion 37 and thereby pressing the O-ring 39 against the side surface of the outer pipe 7 through the pressing member 38.

The cryotherapy probe 3 is attached to the heat absorbing portion 2a of the FPSC2 as follows. That is, the base end 6a of the inner pipe 6 is connected to the heat absorbing portion 2a as it is sandwiched, and in that state, the base end 7a of the outer pipe 7 is airtightly connected to the side wall 34b of the chamber 34 by the vacuum joint 36 so that it may be opened inside the chamber 34. It is to be noted that fixation of the base end 7a of the outer pipe 7 with the vacuum joint 36 is released as well as the ceiling wall 34a of the chamber 34 is opened and fixation of the base end 6a of the inner pipe 6 with the heat absorbing portion 2a is released, whereby the cryotherapy probe 3 can be removed from the heat absorbing portion 2a.

In the heat absorbing portion 2a, embedded are the heater 12 that heats this heat absorbing portion 2a and the temperature sensor 14 that detects a temperature of the heat absorbing portion 2a. The heater 12 is connected to the control unit 20 through the wire 13 airtightly pulled out from the side wall 34b of the chamber 34, and the temperature sensor 14 is connected to the control unit 20 through the wire 15 airtightly pulled out from the side wall 34b of the chamber 34.

In addition, a pipe 42 that communicates with the vacuum pump 31 is connected to the side wall 34b of the chamber 34. The vacuum pump 31 vacuums an inside of the chamber 34. At this time, the gap S between the inner pipe 6 and the outer pipe 7 is simultaneously vacuumed since the base end 7a of the outer pipe 7 is opened inside the chamber 34.

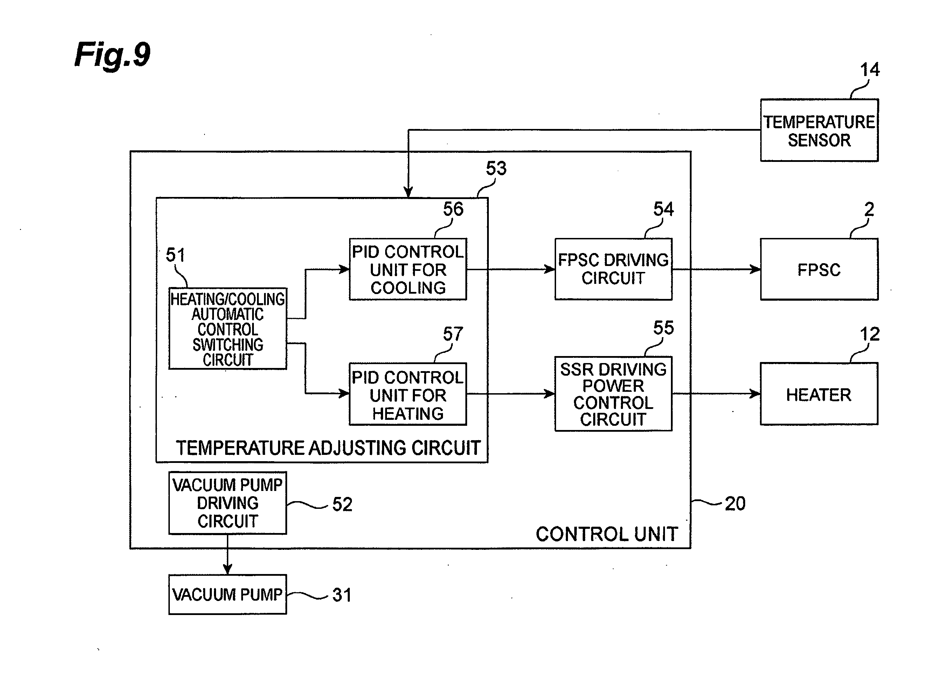

Figure 9 is a block diagram of the control unit 20 of the cryotherapy apparatus 1 of Figure 6. As shown in Figure 9, the control unit 20 has a vacuum pump driving circuit 52 that transmits a drive signal to the vacuum pump 31, a temperature adjusting circuit 53 that obtains a temperature signal showing a temperature detected by the temperature sensor 14, an FPSC driving circuit 54 that transmits a drive signal to the FPSC2, and an SSR driving power control circuit 55 that transmits a drive signal to the heater 12. Further, the temperature adjusting circuit 53 includes a heating/cooling automatic control switching circuit 51, a PID control unit for cooling 56 that instructs the FPSC driving circuit 54 to transmit a drive signal based on an obtained temperature signal, and a PID control unit for heating 57 that instructs the SSR driving power control circuit 55 to transmit a drive signal based on the obtained temperature signal. The heating/cooling automatic control switching circuit 51 controls the PID control unit for cooling 56 and the PID control unit for heating 57 based on input freezing time and heating time. The control unit 20 has independent dedicated temperature control circuits, and determines original allocation of PID (proportion, integration, and differential) in the temperature control to perform the most suitable temperature control since cooling performance of the FPSC2 is different from heating performance of the heater 12, and arrival temperatures thereof also differ from each other.

An example of use of the cryotherapy apparatus 1 configured as described above will be explained with reference to Figure 5.

First, when the cryotherapy probe 3 is attached to the heat absorbing portion 2a of the FPSC2, the vacuum pump 31 is driven, and then the inside of the chamber 34 and the gap S between the inner pipe 6 and the outer pipe 7 are vacuumed. A degree of vacuum required for heat insulation may be approximately 0.1 to 0.01 Pa, and if vacuum is performed with the vacuum pump 31 for low vacuum to medium vacuum to reach a predetermined degree of vacuum, a vacuum valve is closed and the vacuum pump 31 is stopped. It is to be noted that if the pipe 42 of the vacuum pump 31 is separated from the chamber 34 just before an operation after vacuuming, it becomes easy to move the cryotherapy apparatus 1 in an operating room, thus allowing to use this cryotherapy apparatus 1 with a free attitude.

Subsequently, the cryotherapy probe 3 is inserted inside a body in a state where the FPSC2 is supported by the supporting mechanism 4, and the tip of the heat exchanging portion 8 is located on a site with a deep-seated neoplastic lesion under image guidance using MRI, CT, an ultrasonic diagnostic equipment, or the like. In addition, as shown in Figure 5, drive of the FPSC2 (cool drive) and drive of the heater 12 (heat drive) are switched by the control unit 20 based on the temperature detected by the temperature sensor 14 (temperature monitor).

When performing cool drive, the refrigerant encapsulated in the inner pipe 6 is cooled by the heat absorbing portion 2a in the base end 6a in a state of being vacuum-insulated from an outside air, moves to the tip 6b side while cooling the inner pipe 6 changing from vapor to liquid, and thereby the liquefied refrigerant accumulates in the tip 6b. As a result of this, the heat exchanging portion 8 is cooled through the core 32, and an ice ball of approximately 40 millimeters in diameter is formed on the site with the deep-seated neoplastic lesion.

Meanwhile, when performing heat drive, the inner pipe 6 is heated by the heat absorbing portion 2a heated by the heater 12 in the state of being vacuum-insulated from an outside air. As a result of this, the heat exchanging portion 8 is heated, and the ice ball formed on the site with the deep-seated neoplastic lesion is thawed. It is to be noted that the heater 12 works enough to thawe the ice ball as long as a heating value thereof is approximately 50 to 100 W.

As described above, freezing and thawing of the site with the deep-seated neoplastic lesion are repeated (freezing/thawing sequence), and as a result of this, a tumor necrotizes and the site with the deep-seated neoplastic lesion is freeze-treated.

As explained above, with the cryotherapy apparatus 1, similarly to the first embodiment, freezing and thawing can be repeated with respect to a predetermined site inside a body while achieving facilitation of handling, reduction in size, and price-reduction, and it becomes possible not only to freeze-treat the predetermined site more reliably but to exert the following effects.

That is, in the cryotherapy apparatus 1, the base end 6a of the inner pipe 6 of the cryotherapy probe 3 is connected to the heat absorbing portion 2a of the FPSC2 housed in the chamber 34 to be vacuumed, and in that state, the base end 7a of the outer pipe 7 of the cryotherapy probe 3 is airtightly connected to the chamber 34 and is opened therein. In addition, the inside of the chamber 34 and the gap S between the inner pipe 6 and the outer pipe 7 are vacuumed by the vacuum pump 31 connected to the chamber 34 through the pipe 42. As a result of this, as well as the inside of the chamber 34 and the gap S between the inner pipe 6 and the outer pipe 7 can be efficiently vacuumed, the heat absorbing portion 2a and the refrigerant encapsulated in the inner pipe 6 are reliably insulated from the outside (dew condensation or frosting is prevented from generating), and heat exchange performance between the heat absorbing portion 2a and the heat exchanging portion 8 through the inner pipe 6 can be improved by leaps and bounds.

In addition, in the cryotherapy apparatus 1, in view of a situation where the liquefied refrigerant becomes difficult to reach the tip when an internal diameter is reduced, the core 32 made of a material whose heat conductivity is higher than the heat exchanging portion 8 is embedded in the heat exchanging portion 8, and the rear end of the core 32 extends inside the tip 6b of the inner pipe 6. As a result of this, even if the heat exchanging portion 8 is made thinner in order to make it correspond to a predetermined site inside the body, reliable transfer of the heat can be achieved in the heat exchanging portion 8 through the core 32 whose rear end extends inside the tip 6b of the inner pipe 6 in which the refrigerant is encapsulated.

Further, in the cryotherapy apparatus 1, a configuration of the cryotherapy probe 3 can be simplified since the heater 12 and the temperature sensor 14 are embedded in the heat absorbing portion 2a.

The present invention is not limited to the aforementioned first and second embodiments, and it is possible to implement various modifications within the scope of the summary of the invention. For example, in the above-described each embodiment, as a tubular body the cryotherapy probe is used that moves the liquid-phase refrigerant by gravity, but a heat pipe may be used that moves the liquid-phase refrigerant by capillary force. Consequently, it becomes possible to cool a heat exchanging portion regardless of an inclination angle of the tubular body by using the heat pipe instead of the cryotherapy probe as described above. In addition, in the above-described each embodiment, the FPSC is used, but a crank type Stirling cooling machine may be used.

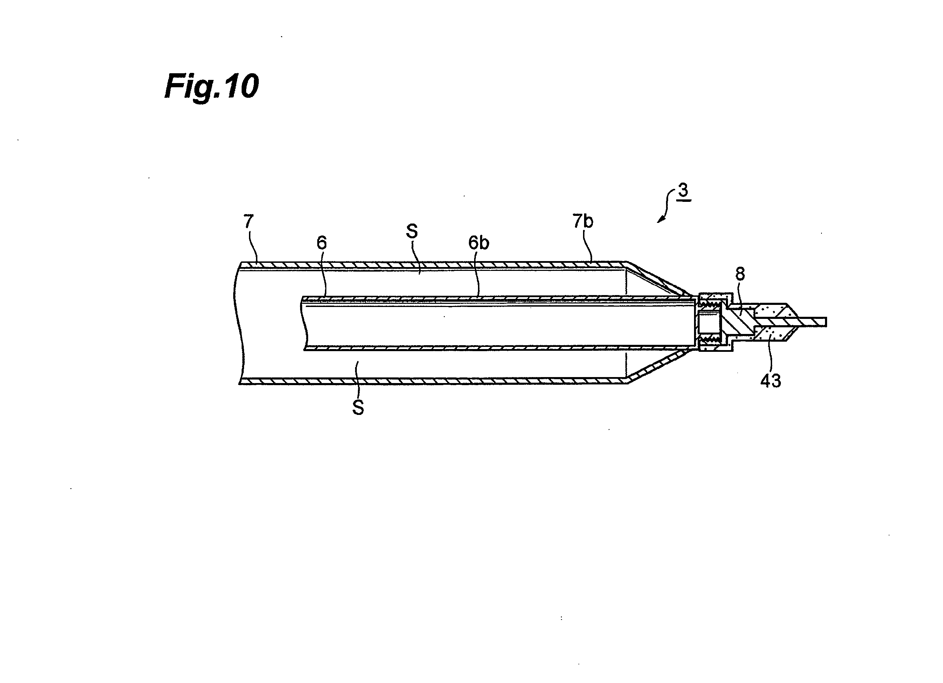

In addition, as shown in Figure 10, the tip 7b of the outer pipe 7 may be airtightly connected to the side surface of the tip 6b of the inner pipe 6 by welding such as brazing as well as providing a male screw portion at the tip 6b of the inner pipe 6, and a female screw portion provided at the rear end of the heat exchanging portion 8 may be screwed to the male screw portion provided at the tip 6b of the inner pipe 6. In this case, depending on a purpose (size, depth, etc. of a site to be treated), a heat insulating cover 43 is removed to replace the heat exchanging portion 8, and a tip shape, a length, etc. of the heat exchanging portion 8 used as a frozen scalpel can be selected.

### Industrial Applicability

The present invention can achieve facilitation of handling, reduction in size, and price-reduction. When ease of handling improves, it becomes possible to achieve reduction of an operation time and reduction of a patient burden in an operation.

## Claims

1. A cryotherapy apparatus for freeze-treating a predetermined site inside a body, comprising:
a Stirling cooling machine; and
a cryotherapy probe attached to a heat absorbing portion of the Stirling cooling machine, **characterized in that**
the cryotherapy probe has:
an inner pipe whose base end is connected to the heat absorbing portion as well as in which a refrigerant that operates below the freezing point is encapsulated;
an outer pipe that covers the inner pipe so that a gap to be vacuumed may be formed; and
a heat exchanging portion provided at a tip of the inner pipe.

2. The cryotherapy apparatus according to claim 1, **characterized by** comprising a heater that heats the heat exchanging portion or the heat absorbing portion.

3. The cryotherapy apparatus according to claim 2, **characterized by** comprising a temperature sensor that detects a temperature of the heat exchanging portion or the heat absorbing portion.

4. The cryotherapy apparatus according to claim 3, **characterized in that**
the Stirling cooling machine is a free piston type Stirling cooling machine, and **characterized by** comprising
a control unit that switches drive of the free piston type Stirling cooling machine and drive of the heater based on a temperature detected by the temperature sensor.

5. The cryotherapy apparatus according to any one of claims 1 to 4, **characterized in that** the heat exchanging portion is removable with respect to the inner pipe.

6. The cryotherapy apparatus according to any one of claims 1 to 5, **characterized in that** the cryotherapy probe is removable with respect to the heat absorbing portion.

7. The cryotherapy apparatus according to any one of claims 1 to 6, **characterized in that** the outer pipe and the heat exchanging portion are made of stainless steel or titanium.

8. The cryotherapy apparatus according to any one of claims 1 to 7, **characterized in that** the Stirling cooling machine is supported by a free-arm supporting mechanism.

9. The cryotherapy apparatus according to any one of claims 1 to 8, **characterized in that**
the heat absorbing portion is housed in a chamber to be vacuumed, and that
the outer pipe is airtightly connected to the chamber and is opened to an inside of the chamber in a state where the base end of the inner pipe is connected to the heat absorbing portion.

10. The cryotherapy apparatus according to claim 9, **characterized by** comprising a vacuum pump that is connected to the chamber, and that vacuums the inside of the chamber and a gap between the inner pipe and the outer pipe.

11. The cryotherapy apparatus according to any one of claims 1 to 10, **characterized in that**
a core made of a material whose heat conductivity is higher than the heat exchanging portion is embedded in the heat exchanging portion, and that
the core extends inside the tip of the inner pipe.

12. A cryotherapy probe used for a cryotherapy apparatus for freeze-treating a predetermined site inside a body, comprising:
an inner pipe in which a refrigerant that operates below the freezing point has been encapsulated;
an outer pipe that covers the inner pipe so that a gap to be vacuumed may be formed; and
a heat exchanging portion provided at a tip of the inner pipe, **characterized in that**
a core made of a material whose heat conductivity is higher than the heat exchanging portion is embedded in the heat exchanging portion, and that
the core extends inside the tip of the inner pipe.
